# EUROPEAN PATENT SPECIFICATION

(11) **EP 3 126 428 B1**
(45) Date of publication and mention of the grant of the patent: **06.02.2019**
(21) Application number: 15718662.8
(22) Date of filing: 27.03.2015
(51) Int. Cl.: C08G 65/34, C08G 65/26, C08G 18/32

(54) **POLYETHER POLYOL PROVIDING GOOD BLOW-GEL BALANCE FOR POLYURETHANE PRODUCTS MADE THEREFROM**
POLYETHERPOLYOL MIT GUTEM BLOW-GEL-GLEICHGEWICHT FÜR DARAUS HERGESTELLTE POLYURETHANPRODUKTE
POLYÉTHER-POLYOL PRÉSENTANT UN BON ÉQUILIBRE SOUFFLAGE-GEL POUR DES PRODUITS EN POLYURÉTHANE PRODUITS À PARTIR DE CELUI-CI

(30) Priority: 01.04.2014 US 201461973328 P
(43) Date of publication of application: 08.02.2017
(73) Proprietor: Dow Global Technologies LLC, Midland, MI 48674 (US)
(72) Inventor: QUINTANILLA, Esther, CH-8048 Zuerich (CH); BANK, David H., Midland, MI 48667 (US); BIRCH, Adrian J., CH-8645 Kempraten-Jona (CH); CASATI, Francois M., CH-8808 Pfaffikon (CH)
(74) Representative: Murgitroyd & Company
(86) International application number: PCT/US2015/022890
(87) International publication number: WO 2015/153316

(56) References cited:
- WO-A1-03/029320
- WO-A1-2010/101700
- WO-A1-2011/106739
- WO-A1-2012/044338
- US-A1- 2011 218 324

## Description

### FIELD OF THE INVENTION

The present invention pertains to a novel tertiary amine initiator and polymeric polyol compositions made therefrom useful for making polyurethane polymers, especially polyurethane foams; said polyurethane polymer foams demonstrating a good balance of mechanical properties, physical properties, and low volatile organic compounds emissions.

### BACKGROUND OF THE INVENTION

Polyether polyols based on the polymerization of alkylene oxides, and/or polyester polyols, are the major components of a polyurethane system together with isocyanates. Polyols can also be filled polyols, such as SAN (styrene/acrylonitrile), PIPA (polyisocyanate polyaddition) or PHD (polyurea) polyols. These systems generally contain additional components such as blowing agents, cross-linkers, chain extenders, surfactants, cell regulators, stabilizers, antioxidants, flame retardant additives, eventually fillers, and typically catalysts such as tertiary amines and/or organometallic salts.

Tertiary amine catalysts generally have a strong odor and many are highly volatile due to their low molecular weight. The release of the tertiary amine during foam processing may present safety and toxicity concerns and the release of residual amine during customer handling is undesirable. The release of tertiary amine catalysts vapor in polyurethane products is also reported to be detrimental to vinyl film and polycarbonate sheets exposed thereto. Fugitive amines are also associated with fogging issues, that is, deposit of solids or liquid film on an automotive wind-shield.

It is desirable to limit the volatility of this amine component or to lessen the amount of its use in a polyurethane formulation. In addition to reducing volatile organic compounds (VOC's), lessening volatility or reducing the level of amine use can reduce worker exposure, improve safety, and address quality concerns.

Compounds with tertiary amine groups are known to be useful catalysts for urethane reactions. Certain polyols, sometimes referred to as an autocatalytic polyols, contain tertiary amine groups which can reduce or eliminate the need for typical tertiary amine catalysts in formulations for polyurethanes. The reactivity of the urethane reaction system can be maintained at a reduced level of tertiary amine catalyst. Further, the presence of multiple tertiary amine groups in such a polyol compound allows it to be chemically bound during a polyurethane gel or foam crosslinking reaction, for example. The resultant product can be substantially free of volatile amine emissions. However, many such autocatalytic polyols if used alone do not provide an optimal blowing:gelling ratio such that polyurethane polymers made therefrom may demonstrate inadequate physical properties and/or mechanical properties. For example, see US Publication No. 2009/0227695 which suggests addition of traditional fugitive type amine catalysts may improve properties. However, such an approach leads to increased emission products.

WO 2011/106739 A1 relates to the reaction of glycerine and glycidol to form a polyglycerine, which is then subsequently reacted with an aminoalcohol. WO 2012/044338 A1 describes a process for producing flexible polyurethane foams from a blend of polyols and an isocyanate. WO 2010/101700 A1 describes a polyol blend for use in the production of polyurethane products. US2011/218324 A1 relates to a process for preparing a polyether alcohol involving a catalyst and carried out in the presence of a polyether alcohol having specified properties. WO 03/029320 A1 describes a process for producing polyurethane products by reacting autocatalytic polyols with a polyisocyanate.

Thus, for urethane applications, especially polyurethane foam applications, it is desirable to produce polymeric polyol compounds or compositions having multiple tertiary amine groups which provide a good blowing:gelling ratio which provide good physical properties and mechanical properties in a polyurethane polymer produced therefrom while reducing, or eliminating, both the amount of fugitive tertiary amine catalyst used and the volatile amine emissions. Accordingly, it is to these ends that the novel tertiary amine initiator of the present invention, and polymeric polyol compositions made therefrom, are directed.

### SUMMARY OF THE INVENTION

The present invention is such a polyol initiator composition comprising the reaction product(s) of:
(i) a dihydroxy tertiary amine compound represented by the structure I: 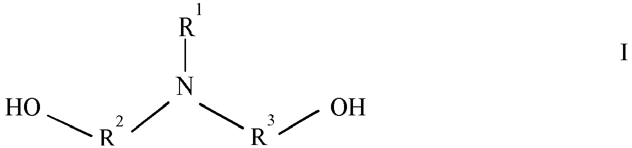 wherein R¹ is a C₁-C₆ linear or branched alkyl group and R² and R³ are independently a C₁-C₆ linear or branched alkyl group and
(ii) a polyhydroxy alcohol, wherein the polyhydroxy alcohol is methylenglycol, diethylenglycol, methylpropylenglycol, dipropyleneglycol, glycerine, trimethylol propane, pentaerythritol, or a sugar,
wherein the mole ratio of dihydroxy tertiary amine compound (i) to the polyhydroxy alcohol (ii) is preferably10:1 to 1:10, preferably R¹ is methyl and R² and R³ are both ethyl and the polyhydroxy alcohol is glycerine for the polyol initiator described herein above.

Another embodiment of the present invention is a first polymeric polyol composition comprising the reaction product of:
(a) the polyol initiator disclosed herein above and
(b) at least one epoxide compound having the structure X: 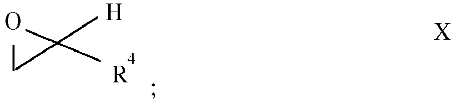 or
   at least one glycidyl ether compound having the structure XI: 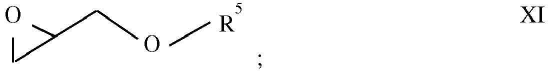 or a combination thereof; wherein R⁴ is hydrogen, phenyl, cyclohexyl, or a C₁-C₁₈ linear or branched alkyl and R⁵ is hydrogen, phenyl, a C₁-C₆ linear or branched alkyl-substituted phenyl, or a C₁-C₁₈ linear or branched alkyl.

Another embodiment of the present invention is a process to make a polyurethane polymer by reaction of a mixture comprising: (A) a polymeric polyol formulation comprising: (i) the first polymeric polyol composition described herein above; (B) at least one organic isocyanate; (C) optionally a blowing agent; and (D) optionally additives or auxiliary agents known per se for the production of polyurethane polymers.

Another embodiment of the present invention is the process described herein above where in the polymeric polyol formulation (A) further comprises: (A)(ii) a second polymeric polyol composition comprising the reaction product of:
(c) a second polyol initiator represented by the following structure XII: 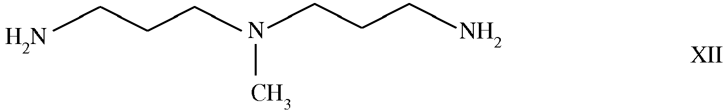 and
(d) at least one epoxide compound having the structure X, at least one glycidyl ether compound having the structure XI, or a combination thereof.

Another embodiment of the present invention is the process described herein above wherein the reaction occurs in the presence of a blowing agent and the polyurethane polymer is produced in the form of a polyurethane flexible foam.

### DETAILED DESCRIPTION OF THE INVENTION

The present invention discloses a novel initiator composition for the production of polyether polyols and polyurethane polymers made therefrom. The initiator composition is the reaction product of a dihydroxy tertiary amine and a polyhydroxy alcohol. Preferably the dihydroxy tertiary amine has the following structure I: 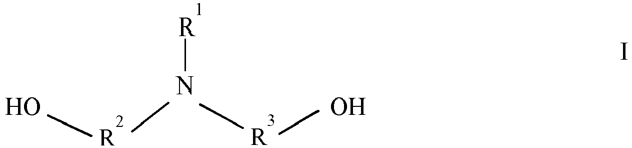 wherein R¹ is a C₁-C₆ linear or branched alkyl group, R² and R³ are independently a C₁-C₆ linear or branched alkyl group. Preferably the dihydroxy tertiary amine is N-methyl diethanolamine (MDEA). The polyhydroxy alcohols are methylenglycol (MEG), diethylenglycol (DEG), methylpropylenglycol (MPG), dipropyleneglycol (DPG), glycerol, trimethylol propane, (TMP), pentaerythritol, and sugars such as sucrose and sorbitol). Most preferred polyhydroxy alcohols are glycerine, glycol, and sugars.

A preferred initiator composition shown in Scheme 1 is the reaction products of MDEA and glycerine: 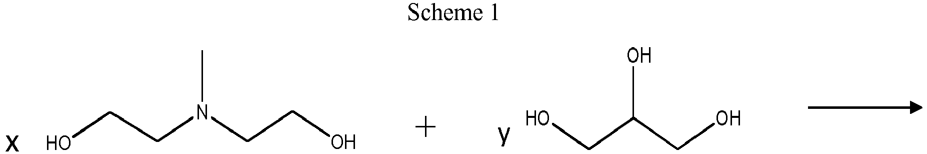 wherein x is preferably an integer of from 1 to 10 and independently y is preferably an integer of from 1 to 10.

The reaction product of the dihydroxy tertiary amine and the polyhydroxy alcohol may comprise a mixture of products as well as partially and/or completely unreacted tertiary amine and/or polyhydroxy alcohol. For example the reaction of N-methyl diethanol amine and glycerine in addition to unreacted N-methyl diethanolamine and/or glycerine, may yield a mixture of products comprising one or more of, but not limited to: 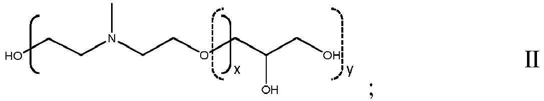 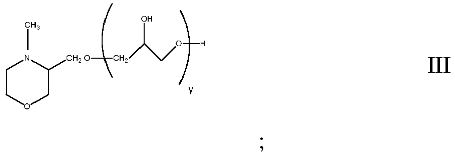 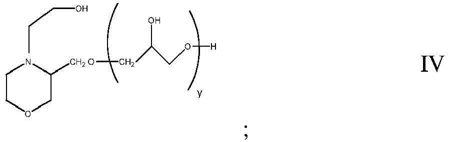 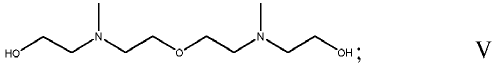 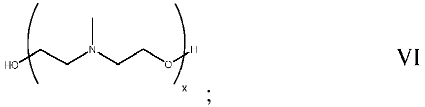 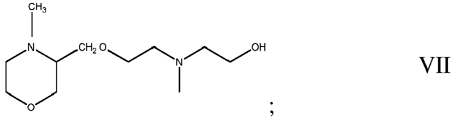 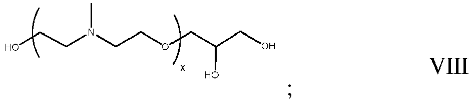 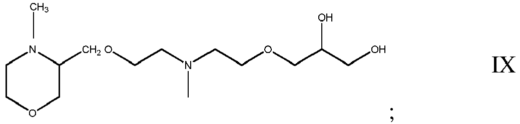 and the like.

The product, i.e., the resulting mixture of one or more reaction product from reacting the tertiary amine and/or polyhydroxy alcohol, may be used to prepare a polymeric polyol composition comprising polyol compounds as produced without any purification or isolation of specific reaction products and/or recovery of unreacted starting materials (e.g., tertiary amine and/or polyhydroxy alcohol). Alternatively, unreacted starting materials may be removed from the reaction products, and/or specific reaction products may be isolated from the reaction product mixture. If specific reaction products are isolated, if desired, they may be further purified.

In one embodiment of the present invention, the mole ratio of the dihydroxy tertiary amine to the polyhydroxy alcohol is from 10:1 to 1:10, preferably 5:1 to 1:5, even more preferably 3:1 to 1:3.

In another embodiment of the present invention, the mole ratio of the dihydroxy tertiary amine to the polyhydroxy alcohol is from 10:1 to 1:1, more preferably from 5:1 to 1:1, even more preferably 3:1 to 1:1.

In another embodiment of the present invention, the mole ratio of the dihydroxy tertiary amine to the polyhydroxy alcohol is from 1:10 to 1:1, more preferably from 1:5 to 1:1, even more preferably 1:3 to 1:1.

A particularly preferred initiator composition is one wherein there are more than one tertiary amine, preferably N-methyl amine, group in the backbone of reaction product between the dihydroxy tertiary amine and polyhydroxy alcohol product. For example, for structure II, x is greater than 1, preferably 2, 3, 4, or 5. N-methyl amine presence may be determined and quantified by Matrix-assisted laser desorption/ionization (MALDI) and Nuclear Magnetic Resonance (NMR) spectroscopy.

The above initiator composition preferably has an average weight average molecular weight (Mw) equal to or less than 5,000, preferably equal to or less than 3,000, more preferably equal to or less than 1,700, and even more preferably equal to or less than 1,000. The above initiator composition preferably have a Mw equal to or greater than 180, preferably equal to or greater than 250, and more preferably equal to or greater than 600. The Mw data in accordance with this disclosure can be determined by Gel Permeation Chromatography.

The above initiator composition preferably has an average hydroxyl number (reported as mg KOH /g) equal to or greater than 34, preferably equal to or greater than 56, more preferably equal to or greater than 99, and even more preferably equal to or greater than 169. The above initiator composition preferably has an average hydroxyl number equal to or less than 935, preferably equal to or less than 674, and more preferably equal to or less than 280. The hydroxyl number is measured by ASTM D4274 D.

The initiator composition comprising the reaction products of the dihydroxy tertiary amine I and a polyhydroxy alcohol as described herein above is useful to produce a novel polymeric polyol composition comprising polymeric polyol compounds. A first polymeric polymer composition is the reaction product(s) of an initiator composition as described herein above with at least one epoxide compound having the structure X: 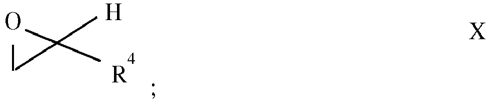 or
at least one glycidyl ether compound having the structure XI: 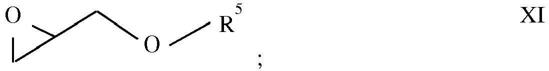 or a combination thereof;
wherein R⁴ is hydrogen, phenyl, cyclohexyl, or a C₁-C₁₈ linear or branched alkyl and
R⁵ is hydrogen, phenyl, a C₁-C₆ linear or branched alkyl-substituted phenyl, or a C₁-C₁₈ linear or branched alkyl.

In formula X, R⁴ can be hydrogen, phenyl, cyclohexyl, or a C₁-C₁₈ linear or branched alkyl. In accordance with one aspect of the present invention, R⁴ is methyl, ethyl, propyl, butyl, pentyl, hexyl, heptyl, 2-ethylhexyl, octyl, decyl, dodecyl, tetradecyl, hexadecyl, or octadecyl. In another aspect, R⁴ can be hydrogen, phenyl, or cyclohexyl. In yet another aspect, R⁴ is hydrogen, methyl, or phenyl. In this aspect, where R⁴ is hydrogen, methyl, butyl, or phenyl, the epoxide compounds of formula X are, respectively, ethylene oxide, propylene oxide, butylene oxide, or styrene oxide.

R⁵ in formula XI can be hydrogen, phenyl, a C₁-C₆ linear or branched alkyl-substituted phenyl, or a C₁-C₁₈ linear or branched alkyl. For instance, R⁵ can be hydrogen, phenyl, or a C₁-C₆ linear or branched alkyl-substituted phenyl, in one aspect of this invention. R⁵ is methyl, ethyl, propyl, butyl, pentyl, hexyl, heptyl, 2-ethylhexyl, octyl, decyl, dodecyl, tetradecyl, hexadecyl, or octadecyl, in another aspect of this invention. Yet, R⁵ can be phenyl or butyl-substituted phenyl in still another aspect of this invention.

The above resulting polymeric polyol composition preferably has a Mw equal to or less than 8,000, preferably equal to or less than 7,000, and even more preferably equal to or less than 6,000. The above resulting polymeric polyol composition preferably have a Mw equal to or greater than 2,000, preferably equal to or greater than 3,000, and more preferably equal to or greater than 4,000.

According to one aspect of the present invention, the polymeric polyol composition disclosed herein above has a hydroxyl number of equal to or less than 800 mg KOH/g, preferably equal to or less than 300, more preferably equal to or less than 170, more preferably equal to or less than 100, and even more preferably equal to or less than 75 mg KOH/g. The resulting polymeric polyol composition disclosed herein above have a hydroxyl number equal to or greater than 15 mg KOH/g, preferably equal to or greater than 20, more preferably equal to or greater than 25, more preferably equal to or greater than 35, and even more preferably equal to or greater than 50 mg KOH/g. Hydroxyl number is determined according to ASTM D 4274.

In another aspect of the present invention, the herein above polymeric polyol composition has an average functionality (F) equal to or less than 8, preferably equal to or less than 7, more preferably equal to or less than 6, more preferably equal to or less than 5, and even more preferably equal to or less than 4. Preferably, the above polymeric polyol composition has an average functionality equal to or greater than 2, and more preferably equal to or greater than 3.

The polymeric polyol compositions of this invention also can be characterized by nitrogen content. For instance, amine values of polymeric polyol compositions disclosed herein above are equal to or less than 5 mg/g, preferably equal to or less than 4, more preferably equal to or less than 3, more preferably equal to or less than 2 mg KOH/g. Nitrogen content is determined according to ASTM D 6979 and reported as percent Nitrogen.

Another suitable polymeric polyol composition for use in making a polyurethane polymer of the present invention is one resulting from the reaction product of at least one epoxide compound having the structure X disclosed herein above, at least one glycidyl ether compound having the structure XI disclosed herein above, or a combination thereof with an initiator represented by the following structure XII:

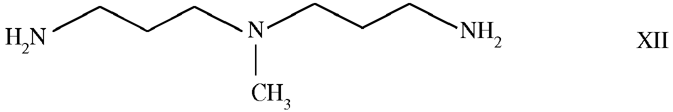

The above resulting polymeric polyol composition comprising initiator XII preferably has a Mw equal to or less than 20,000, preferably equal to or less than 15,000, more preferably equal to or less than 10,000, more preferably equal to or less than 8,000, more preferably equal to or less than 6,000, more preferably equal to or less than 5500, more preferably equal to or less than 5,000, and even more preferably equal to or less than 4,500. The above resulting polymeric polyol composition preferably have a Mw equal to or greater than 400, preferably equal to or greater than 500, preferably equal to or greater than 600, more preferably equal to or greater than 700, more preferably equal to or greater than 800, preferably equal to or greater than 900, preferably equal to or greater than 1,000, and even preferably equal to or greater than 1,500. The Mw data in accordance with this disclosure, and the data presented in the Examples that follow, can be determined by Gel Permeation Chromatography.

According to one aspect of the present invention, the polymeric polyol composition disclosed herein above has a hydroxyl number equal to or less than 600 mg KOH/g, preferably equal to or less than 550, more preferably equal to or less than 500, more preferably equal to or less than 450, and even more preferably equal to or less than 400 mg KOH/g. The resulting polymeric polyol composition disclosed herein above have a hydroxyl number equal to or greater than 5 mg KOH/g, preferably equal to or greater than 10, more preferably equal to or greater than 15 , more preferably equal to or greater than 20, and even more preferably equal to or greater than 30 mg KOH/g.

In another aspect of the present invention, the herein above polymeric polyol composition can have a hydroxyl equivalent weight (EW) equal to or less than 12,000, preferably equal to or less than 10,000, more preferably equal to or less than 6,000, more preferably equal to or less than 4,000, and even more preferably equal to or less than 2,000. The herein above resulting polymeric polyol composition have a hydroxyl equivalent weight equal to or greater than 75, preferably equal to or greater than 100, more preferably equal to or greater than 125, more preferably equal to or greater than 150, and even more preferably equal to or greater than 200.

Polymeric polyol compositions of this invention also can be characterized by an amine value. For instance, amine values of polymeric polyol compositions disclosed herein above are equal to or less than 800 mg KOH/g, preferably equal to or less than 700, more preferably equal to or less than 600, and even more preferably equal to or less than 500 mg KOH/g. The amine values of polymeric polyol compositions disclosed herein above are equal to or greater than 10 mg KOH/g, preferably equal to or greater than 25, more preferably equal to or greater than 50, and even more preferably equal to or greater than 75 mg KOH/g.

Making polymeric polyol compositions is well known in the art; any suitable process to make polymeric polyol compositions from initiator I and/or initiator XII above is acceptable. For instance, initiator I and/or initiator XII can be mixed with a catalyst and this mixture is subsequently reacted with ethylene oxide or propylene oxide at a temperature in a range from about 100°C to 160°C. A traditional catalyst used in this reaction, and known to those of skill in the art, is KOH. Other alkaline hydroxide or hydroxide hydrate catalysts based on Ba (barium) or Sr (strontium) can be employed as the alkoxylation catalyst; producing products with less unsaturation than those produced using the traditional KOH catalyst. Processes for producing polyols using Ba or Sr catalysts are described in USP 5,070,125; 5,010,187; and 5,114,619. High levels of unsaturation, especially with polyols of high equivalent weight, act as chain terminators in polyurethane foam production, giving rise to, for example, foams with poor compressive strength, poor tensile strength, reduced reactivity, and reduced aging performance under humid conditions. The Ba and Sr catalysts also provide improved primary hydroxyl capping efficiency for the same weight percentage of ethylene oxide used. When using Ba or Sr catalysts, water can be added during the reaction of the ethylene oxide or propylene oxide with the initiator. This water addition can reduce the amount of unsaturation in the final polyol product. Another catalyst that can be used to produce polyols is a double metal cyanide (DMC) catalyst, which may provide a different molecular weight distribution of the polymeric polyol composition from that achieved using KOH. Examples of double metal cyanide catalysts are provided in USP 5,470,813; 5,589,431; and 6,008,263.

We have found that a polymeric polyol composition comprising one or more polymeric polyol composition based on initiator I is particularly useful for making polyurethane polymers, especially polyurethane foam polymers having good mechanical properties, good physical properties, and having low emission products. Further, we have found that a polymeric polyol formulation comprising one or more first polymeric polyol composition based on initiator I and one or more additional polymeric polyol composition is particularly useful for making polyurethane polymers, especially polyurethane foam polymers having good mechanical properties, good physical properties, and having low emission products. Moreover, we have found that a polymeric polyol formulation comprising one or more polymeric polyol composition based on initiator I and one or more polymeric polyol composition comprising initiator XII is particularly useful for making polyurethane polymers, especially polyurethane foam polymers having good mechanical properties, good physical properties, and having low emission products.

In a polyol formulation, wherein more than one polymeric polyol composition is used, the weight ratio of the first polymeric polyol composition to the at least one second polyol can range from 50:1 to 1:5,000. In other aspects, the weight ratio of the first polymeric polyol composition to the at least one second polyol in the polyol formulation can range from 10:1 to 1:1,000, from 5:1 to 1:500, or from 4:1 to 1:250. Yet, in other aspect, the weight ratio of the first polymeric polyol composition to the at least one second polyol is in a range from 3:1 to 1:100, or from 2:1 to 1:50.

While compositions and methods are described in terms of "comprising" various components or steps, the compositions and methods can also "consist essentially of" or "consist of" the various components or steps.

Generally, polyurethane foam catalyst systems comprise compounds which accelerate both the blowing (water-isocyanate) and gelling (polyol-isocyanate) reactions. It is beneficial to balance these reactions in order to produce quality foams with acceptable properties. Compositions and formulations of the present invention can comprise a single compound which accelerates, but keeps in balance, both the blowing and gelling reactions. Alternatively, the compositions can comprise at least one catalyst that predominantly accelerates the blowing reaction (a blowing catalyst), or at least one catalyst that predominantly accelerates the gelling reaction (a gelling catalyst), or a combination thereof. As described herein, a blowing catalyst is a catalyst that predominantly accelerates the blowing reaction, but can also, in certain circumstances, accelerate the gelling reaction, albeit to a lesser degree. Similarly, a gelling catalyst is a catalyst that predominantly accelerates the gelling reaction, but can also, in certain circumstances, accelerate the blowing reaction, albeit to a lesser degree. Surprisingly, we have found that a polymeric polyol formulation comprising one or more polymeric polyol composition based on initiator I and one or more polymeric polyol composition comprising initiator XII provides a good blowing:gelling ratio such that polyurethane polymers made form said mixture of polymeric polyols, especially polyurethane foam polymers, have very good mechanical properties and physical properties and demonstrate low levels of emission products.

The presence of multiple tertiary amine groups in the polymeric polyol compositions of the present invention can either reduce or eliminate the need to include a conventional fugitive urethane catalyst when formulating a polyurethane polymer or polyurethane polymer foam. However, in other aspects of the present invention, conventional urethane catalysts can be employed in compositions or formulations along with such polymeric polyol compositions.

In addition to the polymeric polyol compositions disclosed herein above, one or more additional polyol may be used in the polymeric polyol formulation for use in making a polyurethane polymer of the invention. As used herein the term polyols are those materials having at least one group containing an active hydrogen atom capable of undergoing reaction with an isocyanate. Preferred among such compounds are materials having at least two hydroxyls, primary or secondary, or at least two amines, primary or secondary, carboxylic acid, or thiol groups per molecule. Compounds having at least two hydroxyl groups or at least two amine groups per molecule are especially preferred due to their desirable reactivity with polyisocyanates.

Suitable polyols that may be used to produce polyurethane foams of the present invention are well known in the art and include those described herein and any other commercially available polyol and/or SAN, PIPA or PHD copolymer polyols. Such polyols are described in "Polyurethane Handbook", by G. Oertel, Hanser publishers. Mixtures of one or more polyols and/or one or more copolymer polyols may also be used to produce polyurethane products according to the present invention.

Representative polyols include polyether polyols, polyester polyols, polyhydroxy-terminated acetal resins, hydroxyl-terminated amines and polyamines. Natural oil based polyols can also be used. Examples of these and other suitable isocyanate-reactive materials are described more fully in USP 4,394,491. Alternative polyols that may be used include polyalkylene carbonate-based polyols and polyphosphate-based polyols. Preferred are polyols prepared by adding an alkylene oxide, such as ethylene oxide, propylene oxide, butylene oxide or a combination thereof, to an initiator or blend of initiators to give a final polyol a nominal functionality having from 2 to 8, preferably 2 to 6 active more preferably 2.1 to 4 active hydrogen atoms. Catalysis for this polymerization can be either anionic or cationic, with catalysts such as KOH, CsOH, boron trifluoride, or a double cyanide complex (DMC) catalyst such as zinc hexacyanocobaltate, or quaternary phosphazenium compounds. In the case of alkaline catalysts, these are eliminated from the polyol at the end of production by a proper finishing step, such as coalescence, magnesium silicate (magsil) separation, ion exchange or less preferably by acid neutralization. In the case of DMC catalyst produced polyols, the finishing step can be avoided.

The polyols or blends thereof employed depend upon the end use of the polyurethane foam to be produced. The hydroxyl number and molecular weight of the polyol or polyols employed can vary accordingly over a wide range. In general, the hydroxyl number of the polyols employed for use in producing a flexible or visco-elastic foam may range from 15 to 300.

In the production of a flexible polyurethane foam, the polyol is preferably a polyether polyol and/or a polyester polyol or a polyetherester polyol. The polyol generally has an average functionality ranging from 2 to 5, preferably 2 to 4, and an average hydroxyl number ranging from 15 to 300 mg KOH/g, preferably from 20 to 200, and more preferably from 20 to 70mg KOH/g. As a further refinement, the specific foam application will likewise influence the choice of base polyol. As an example, for molded foam, the hydroxyl number of the base polyol may be on the order of 20 to 60 with ethylene oxide (EO) capping, and for slabstock foams the hydroxyl number may be on the order of 25 to 75 and is either mixed feed EO/PO (propylene oxide) or is only slightly capped with EO or is 100 percent PO based.

In the production of a visco-elastic foam, polyols having a functionality as for flexible foam can be used, however; the polyol or polyol blend will preferably contain polyols having a hydroxyl number from 150 to 300 mg KOH/g. For the production of semi-rigid foams or microcellular elastomers, it is preferred to use a trifunctional polyol with a hydroxyl number of 30 to 80.

The isocyanates which may be used in the present invention include aliphatic, cycloaliphatic, arylaliphatic and aromatic isocyanates. For the production of slabstock foam, aromatic isocyanates are preferred.

Examples of suitable aromatic isocyanates include the 4,4'-, 2,4' and 2,2'-isomers of diphenylmethane diisocyante (MDI), blends thereof and polymeric and monomeric MDI blends toluene-2,4- and 2,6-diisocyanates (TDI), m- and p-phenylenediisocyanate, chlorophenylene-2,4-diisocyanate, diphenylene-4,4'-diisocyanate, 4,4'-diisocyanate-3,3'-dimehtyldiphenyl, 3-methyldiphenyl-methane-4,4'-diisocyanate and diphenyletherdiisocyanate and 2,4,6-triisocyanatotoluene and 2,4,4'-triisocyanatodiphenylether.

Mixtures of isocyanates may be used, such as the commercially available mixtures of 2,4- and 2,6-isomers of toluene diisocyantes. A crude polyisocyanate may also be used in the practice of this invention, such as crude toluene diisocyanate obtained by the phosgenation of a mixture of toluene diamine or the crude diphenylmethane diisocyanate obtained by the phosgenation of crude methylene diphenylamine. TDI/MDI blends may also be used. MDI or TDI based prepolymers can also be used, made either with a polymeric polyol composition comprising initiator (I) and/or , a polymeric polyol composition comprising initiator (XII) or any other polyol as described heretofore. Isocyanate-terminated prepolymers are prepared by reacting an excess of polyisocyanate with polyols, including aminated polyols or imines/enamines thereof, or polyamines.

Examples of aliphatic polyisocyanates include ethylene diisocyanate, 1,6-hexamethylene diisocyanate (HDI), isophorone diisocyanate (IPDI), cyclohexane 1,4-diisocyanate, 4,4'-dicyclohexylmethane diisocyanate (H₁₂MDI), saturated analogues of the above mentioned aromatic isocyanates and mixtures thereof.

For the production of flexible foams, the preferred polyisocyanates are the toluene-2,4- and 2,6-diisocyanates or MDI or combinations of TDI/MDI or prepolymers made therefrom.

For producing a polyurethane-based foam, a blowing agent is generally required. In the production of flexible polyurethane foams, water is preferred as the blowing agent. The amount of water is preferably in the range of from 0.5 to 10 parts by weight, more preferably from 2 to 7 parts by weight based on 100 parts by weight of the polyol and even more preferably the water is between 2 and 5 parts per hundred parts polyol. In some applications the water is preferably present in 3 parts by weight of the polyol. In some preferred embodiment, the water is present at of 6 parts or less by weight of the polyol. When water is present at less than 3 parts, a small conventional amine catalyst can be used to enhance the reactivity of the system. The amount of conventional amine catalyst included in such formulations is generally from 0.01 to 0.1 weight percent of the polyol. To further reduce the level of volatile amine, such a catalyst is used as less than 0.05 weight percent of the polyol. Although not preferred, other blowing agents can be liquid or gaseous carbon dioxide, methylene chloride, acetone, pentane, isopentane, cyclopentane, methylal or dimethoxymethane, dimethylcarbonate. Use of artificially reduced, or increased, atmospheric pressure, such as disclosed in US 5,194,453, or frothing, can also be contemplated with the present invention.

In addition to the foregoing critical components, it is often desirable to employ certain other ingredients in preparing polyurethane polymers. Among these additional ingredients are catalysts, surfactants, preservatives, flame retardants, colorants, antioxidants, reinforcing agents, stabilizers and fillers, recycled polyurethane powder.

One or more catalysts for the reaction of the polyol with the polyisocyanate can be used. Any suitable urethane catalyst may be used, including tertiary amine compounds, amines with isocyanate reactive groups and organometallic compounds. Preferably the reaction is carried out in the absence of an amine or an organometallic catalyst or a reduced amount as described above. Exemplary tertiary amine catalysts include triethylenediamine; N-methylmorpholine; N,N-dimethylcyclohexylamine; pentamethyldiethylenetriamine; tetramethylethylenediamine; bis (dimethylaminoethyl)ether; N-ethylmorpholine; dimethylethanolamine; N-cocomorpholine; 1-methyl-4-dimethylaminoethyl-piperazine; 3-methoxy-N-dimethylpropylamine; N,N-dimethyl-N',N'-dimethyl isopropylpropylenediamine; N,N-diethyl-3-diethylaminopropylamine and dimethylbenzylamine. Exemplary organometallic catalysts include organomercury, organolead, organoferric and organotin catalysts, with organotin catalysts being preferred among these. Suitable tin catalysts include stannous chloride, tin salts of carboxylic acids such as dibutyltin di-laurate, and stannous octoate, as well as other organometallic compounds such as are disclosed in U.S. Patent 2,846,408. A catalyst for the trimerization of polyisocyanates, resulting in a polyisocyanurate, such as an alkali metal alkoxide may also optionally be employed herein. The amount of amine catalysts can vary from 0.02 to 5 percent in the formulation or organometallic catalysts from 0.001 to 1 percent in the formulation can be used.

In one preferred embodiment of the present invention, the foams are produced with a catalyst package that includes a tin catalyst. Preferably such formulations do not contain a conventional amine catalyst.

In making polyurethane foam, it is generally preferred to employ an amount of a surfactant to stabilize the foaming reaction mixture until it cures. Such surfactants advantageously comprise a liquid or solid organosilicone surfactant. Other surfactants include polyethylene glycol ethers of long-chain alcohols, tertiary amine or alkanolamine salts of long-chain alkyl acid sulfate esters, alkyl sulfonic esters and alkyl arylsulfonic acids. Such surfactants are employed in amounts sufficient to stabilize the foaming reaction mixture against collapse and the formation of large, uneven cells. Typically, 0.2 to 3 parts of the surfactant per 100 parts by weight total polyol (b) are sufficient for this purpose.

A crosslinking agent or a chain extender may be added, if necessary. The crosslinking agent or the chain extender includes low-molecular weight polyhydric alcohols such as ethylene glycol, diethylene glycol, 1,4-butanediol, and glycerin; low-molecular weight amine polyol such as diethanolamine and triethanolamine; polyamines such as ethylene diamine, xlylenediamine, and methylene-bis(o-chloroaniline). The use of such crosslinking agents or chain extenders is known in the art as disclosed in U.S. Patents 4,863,979, 4,883,825 and 4,963,399 and EP 549,120.

When preparing foams for use in transportation, a flame retardant is sometimes included as an additive. Any known liquid or solid flame retardant can be used with the autocatalytic polyols of the present invention. Generally such flame retardant agents are halogen-substituted phosphates and inorganic flame proofing agents. Common halogen-substituted phosphates are tricresyl phosphate, tris(1,3-dichloropropyl phosphate, tris(2,3-dibromopropyl) phosphate and tetrakis (2-chloroethyl)ethylene diphosphate. Inorganic flame retardants include red phosphorous, aluminum oxide hydrate, antimony trioxide, ammonium sulfate, expandable graphite, urea or melamine cyanurate or mixtures of at least two flame retardants. In general, when present, flame retardants are added at a level of from 5 to 50 parts by weight, preferable from 5 to 25 parts by weight of the flame retardant per 100 parts per weight of the total polyol present.

The particular polyol, polyol mixture, and polyol amount used in the polyurethane foam forming composition can be chosen based on the factors such as the desired polyurethane foam properties and/or the particular end use of the foam product. Properties of the polyol such as molecular weight or hydroxyl number can be chosen to provide foam characteristics selected from: low density, high density foam, conventional, high resilient, hot molding, cold molding, flexible, and rigid, and desired combinations thereof. For many applications or foam properties, the hydroxyl number of the polyol is in the range of about 15 to about 800.

Compositions for the production of flexible polyurethane foams typically include a polyether polyol and/or a polyester polyol. The polyol generally has an average functionality ranging from 2 to 5, preferably 2 to 4, and an average hydroxyl number ranging from 20 to 100 mg KOH/g, preferably from 20 to 75 mgKOH/g (see, for example, USP 7,361,695).

For molded foam, the hydroxyl number of the base polyol can be in the range of about 20 to about 60 with ethylene oxide (EO) capping, and for slabstock foams the hydroxyl number can be in the range of about 25 to about 75 (see, for example, USP 7,361,695).

Processes for producing polyurethane foam products are well known in the art. In general components of the polyurethane-forming reaction mixture can be mixed together in any convenient manner, for example by using any of the mixing equipment described in the prior art such as in Polyurethane Handbook, by G. Oertel, Hanser publisher.

The polyurethane products can be produced continuously or discontinuously, by injection, pouring, spraying, casting, calendering, etc. Foams can be made under free rise or molded conditions, at atmospheric pressure, reduced or increased air pressure, with or without release agents, in-mold coating, or with any inserts or skin put in the mold. Flexible molded foams can be mono- or dual-hardness.

For example, a polyurethane polymer of the present invention may be made by the reaction of a mixture comprising: a polymeric polyol formulation comprising: a polymeric polyol composition comprising initiator (I); at least one organic isocyanate; optionally a blowing agent; and optionally additives or auxiliary agents known per se for the production of polyurethane polymers for example, catalysts, surfactants, preservatives, flame retardants, colorants, antioxidants, reinforcing agents, stabilizers, fillers, and recycled polyurethane powder

Slabstock foam is conveniently prepared by mixing the foam ingredients and dispensing them into a trough or other region where the reaction mixture reacts, rises freely against the atmosphere (sometimes under a film or other flexible covering) and cures. In common commercial scale slabstock foam production, the foam ingredients (or various mixtures thereof) are pumped independently to a mixing head where they are mixed and dispensed onto a conveyor that is lined with paper or plastic. Foaming and curing occurs on the conveyor to form a foam bun. The resulting foams are typically from about from 10 kg/m³ to a maximum of 80 kg/m³. A preferred range is from 10 kg/m³ to 70 kg/m³ and more preferably from 10 kg/m³ to 60 kg/m³ in density. In an even more preferred embodiment the slabstock foam has a density of 40 kg/m³ or less.

A preferred slabstock foam formulation contains from 3 to 6, preferably 4 to 5 parts by weight water are used per 100 parts by weight high equivalent weight polyol at atmospheric pressure. At reduced or increased pressure these levels are adjusted accordingly to obtain targeted densities, i.e., reduced pressure will generally decrease the density.

Polyurethane foams produced using polymeric polyol compositions of the invention can be used in applications known in the industry. For example, flexible, semi-flexible foams and find use in applications such as vehicle applications such as seats, sun visors, armrests, door panels, noise and heat insulation parts, dashboards, or instrument panels. Exemplary placement of the foams includes locations such as under the carpet or in other parts of the car interior or in the engine compartment. Foam of the invention can also be used in many domestic applications such as shoe soles, cloth interliners, appliance, furniture, and bedding.

The polyurethane foams of the present invention may optionally be characterized by one or more foam properties, including, but not limited to density, indentation force deflection (IFD), sag factor, recovery ratio, guide factor, compression load deflection (CLD), percent compression set, tensile strength, elongation, and tear strength.

Density is weight per unit volume (weight/volume) and typically expressed as lbs/ft3 (pcf) or g/L. Exemplary densities are in the range of about 20 g/L to about 80 g/L, or more specifically in the range of about 25 g/L to about 32 g/L.

Compression force deflection (CFD), such as measured by the ISO 3386/1 standard, is a testing standard designed to measure the compression stress/strain (load divided by specimen surface area at a certain compression percentage) characteristic of foam. CFD is also a measure of firmness and is expressed in pounds per square inch (psi), at a given percentage deflection. Exemplary densities are in the range of about 20 g/L to about 80 g/L, or more specifically in the range of about 25 g/L to about 32 g/L.

Percent compression set (CS), such as measured by the ISO 1856 standard, is a measure of the permanent deformation of a foam after it has been compressed between two metal plates for a controlled time period and temperature condition. The standard conditions are 22 hours at 70°C (158°F). Exemplary compression set values are in the range of about 1 to about 20, or more specifically in the range of about 5 to about 7.

Tensile properties is a measure according to ISO 1798 and tensile strength is the amount of force required to break an area of foam as it is pulled apart, and is generally expressed in pounds per square inch (psi). Foam compositions can be prepared to provide foam with a desired tensile strength, or a tensile strength within a desired range.

Elongation, such as measured by the ISO 1798 standard, is a measure of the extent to which the foam can be stretched before it breaks and is expressed as a percentage of its original length. Elongation is measured at the same time, as tensile strength is determined; therefore, the sample size is the same. Exemplary elongation values are in the range of about 50 to about 200, or more specifically in the range of about 110 to about 130.

Tear strength, such as measured by the ASTM D3574 standard, is a measure of the force required to continue a tear in foam after a split has been started and is expressed in pounds per linear inch (pli). Exemplary tear strengths are in the range of about 50 to about 350, or more specifically in the range of about 195 to about 230.

Emissions Measurements are done following VDA 278 (*Thermal Desorption Analysis of Organic Emissions for the Characterization of Non-Metallic Materials for Automobiles)* official protocol: - VOC value: volatile organic compounds (90°C, 30 min); Emission limits depend on car manufacturer, e.g., Daimler VOC ≤ 100 µg/g

The following examples are given to illustrate the invention and should not be interpreted as limiting in anyway. Unless stated otherwise, all parts and percentages are given by weight.

### EXAMPLES

A description of the raw materials used in the examples is as follows.

| | |
|---|---|
| SPECFLEX™ NC 701.01 | is a grafted polyether polyol by copolymerization of styrene and acrylonitrile available from The Dow Chemical Company. |
| SPECFLEX NC 632 | is a 1,700 equivalent eight polyoxypropylene polyoxyethylene polyol initiated with a blend of glycerol and sorbitol available from The Dow Chemical Company. |
| VORACTIV™ NC779 | is amine initiated polyether polyol having a hydroxy number of 33 mg KOH/g available from The Dow Chemical Company. |
| NIAX™ A-1 | is a 70 percent bis(2-dimethylaminoethyl) ether, diluted with 30 weight percent of dipropylene glycol from Momentive Performance Materials. |
| DABCO™ 33 LV | is a 33 wt% solution of triethylenediamine in propylene glycol available from Air Products and Chemicals Inc. |
| DABCO NE 1070 | is a reactive non-emissive amine catalysis available from Air Products. |
| DABCO NE 300 | is a reactive non-emissive amine catalysis available from Air Products. |
| DEOA | is 85% diethanolamine in water. |
| Glycerine | is industrial grade. |
| TEGOSTAB™ B-8736 | is a silicone surfactant available from Evonik Industries. |
| H₂O | is distilled water. |
| VORANATE™ T-80 | is an 80:20 TDI mixture of 2,4 to 2,6 isomers available from The Dow Chemical Company. |

Properties are determined as follows:

| | |
|---|---|
| Viscosity | is determined according to ASTM D 4878. |
| VOC | amines in VOC's determined according to VDA 278. |
| Aging CS | is aging compression set determined according to ISO 1856 |

### Example 1

### Synthesis of an initiator composition that is the reaction product of a dihydroxy tertiary amine and a polyhydroxy alcohol

1967 g of N-methyl diethanolamine (MDEA), 25.8 g Na₂CO₃, and 3055 g of glycerine are loaded in a reactor adapted for high temperature reactions. The water formed due to the polycondensation is collected in an external cold trap in dry ice. MDEA and traces of glycerine are as well found in the trap during the condensation reaction. The non reacted monomers are recycled two times by reloading them back into the reactor. The reaction is allowed to progress for a total of 20 hours: 5 hours at is 190°C, then 7 hours at 210°C, and then 8 hours at 230°C. At the conclusion of 20 hours, the non reacted MDEA is stripped from the reactor using a nitrogen purge and the reaction product is not further finished. The reaction product has a hydroxyl number of 793 mg/g, 0.376 percent water, total unsaturation of 0.181 meq/g, 14630 ppm Na, and a pH of 8.7.

### Example 2

### Synthesis of an ethylene oxide capped polyoxypropylene polyol from the initiator composition of Example 1

The reaction product of MDEA and glycerine described herein above in Example 1 is alkoxylated with propylene oxide and 3 percent KOH and allowed to digest for 3 hours at 115°C and capped with 18 percent ethylene oxide at 130°C with a final digestion of 30 minutes at 115°C. The product is filtered finished adding 500 ppm of IRGANOX™ 1076. 4500 g of the polyol is obtained having a Mw of 4800, a hydroxyl number of 41.7 mg/g, total unsaturation of 0.094 meq/g, and a viscosity at 25°C of 804 cSt.

### Comparative Examples A to C and Examples 3 and 4 Hand-mixed foams using the formulations given in Table I.

Hand-mixed foams are made by preblending polyols, water, surfactant, catalysts; the isocyanate is added to the polyol blend and the mixture stirred for 5 seconds at 2,000 RPM. Foaming parameters are determined by pouring the reactants into a 20 x 20 x 20 cardboard box and allowed to free rise. Foam properties and emissions are derived from foams molded in a square mold (9 liters) set up at temperature 65°C.

**Table I**

| Comparative Example | A | | B | C | |
|---|---|---|---|---|---|
| Example | | 3 | | | 4 |
| COMPONENTS | | | | | |
| SPECFLEX NC 701.01 | 40 | 40 | 40 | 40 | 40 |
| SPECFLEX NC 632 | 60 | 15 | 60 | 40 | 40 |
| Example 2 | | 20 | | | 20 |
| VORACTIV NC779 | | 20 | | 20 | |
| DABCO 33LV | 0.35 | | | | |
| NAIX A1 | 0.1 | | | | |
| DABCO NE 1070 | | | 0.6 | | |
| DABCO NE 300 | | | 0.3 | | |
| DEOA | 0.7 | 0.7 | 0.7 | 0.7 | 0.7 |
| Glycerine | 0.5 | 0.5 | 0.5 | 0.5 | 0.5 |
| TEGOSTAB B8736 | 0.6 | 0.6 | 0.6 | 0.6 | 0.6 |
| H₂O | 3.2 | 3.2 | 3.2 | 3.2 | 3.2 |
| VORANATET-80 | 100 | 100 | 100 | 100 | 100 |

| PROPERTIES | | | | | |
|---|---|---|---|---|---|
| Gelling Time, s | 74 | 80 | 85 | did not gel | 114 |
| Amines in VOC's, µg/g | 474 | 0-2 | 15.4 | na | 9 |
| Aging CS, % | 10-15 | 10-15 | 30-35 | na | 10-15 |

## Claims

1. A polyol initiator composition comprising the reaction product(s) of:
(i) a dihydroxy tertiary amine compound represented by the structure I: 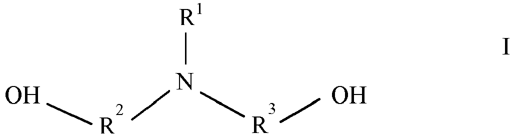
wherein R¹ is a C₁-C₆ linear or branched alkyl group and
R² and R³ are independently a C₁-C₆ linear or branched alkyl group and
(ii) a polyhydroxy alcohol, wherein the polyhydroxy alcohol is methylenglycol, diethylenglycol, methylpropylenglycol, dipropyleneglycol, glycerine, trimethylol propane, pentaerythritol, or a sugar.

2. The polyol initiator of Claim 1 wherein the mole ratio of dihydroxy tertiary amine compound to the polyhydroxy alcohol is 10:1 to 1:10.

3. The polyol initiator of Claims 1 or 2 wherein R¹ is methyl and R² and R³ are both ethyl and the polyhydroxy alcohol is glycerine.

4. A first polymeric polyol composition comprising the reaction product of:
(a) the polyol initiator of Claims 1, 2 or 3 and
(b) at least one epoxide compound having the structure X: 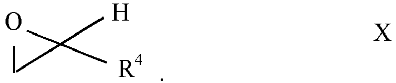 or
at least one glycidyl ether compound having the structure XI: 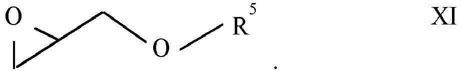 or a combination thereof;
wherein R⁴ is hydrogen, phenyl, cyclohexyl, or a C₁-C₁₈ linear or branched alkyl and
R⁵ is hydrogen, phenyl, a C₁-C₆ linear or branched alkyl-substituted phenyl, or a C₁-C₁₈ linear or branched alkyl.

5. A process to make a polyurethane polymer by reaction of a mixture comprising:
(A) a polymeric polyol formulation comprising:
(i) the first polymeric polyol composition of Claim 4;
(B) at least one organic isocyanate;
(C) optionally a blowing agent;
and
(D) optionally additives or auxiliary agents known per se for the production of polyurethane polymers.

6. The process of Claim 5 wherein the polymeric polyol formulation (A) further comprises:
(A)(ii) a second polymeric polyol composition comprising the reaction product of:
(c) a second polyol initiator represented by the following structure XII: 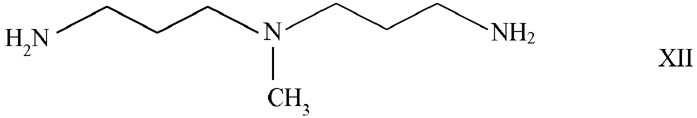 and
(d) at least one epoxide compound having the structure X, at least one glycidyl ether compound having the structure XI, or a combination thereof.

7. The process of Claims 5 or 6 wherein the reaction occurs in the presence of a blowing agent and the polyurethane polymer is produced in the form of a polyurethane flexible foam.

## Patentansprüche

1. Eine Polyolinitiatorzusammensetzung, beinhaltend das/die Reaktionsprodukt(e) von Folgendem:
(i) einer tertiären Dihydroxyaminverbindung, die durch die Struktur I dargestellt ist: 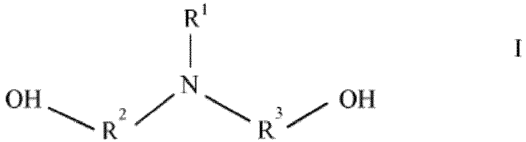
wobei R¹ eine lineare oder verzweigte C₁-C₆-Alkylgruppe ist
und
R² und R³ unabhängig eine lineare oder verzweigte C₁-C₆-Alkylgruppe sind, und
(ii) einem Polyhydroxyalkohol, wobei der Polyhydroxyalkohol Methylenglykol, Diethylenglykol, Methylpropylenglykol, Dipropylenglykol, Glycerin, Trimethylolpropan, Pentaerythrit oder ein Zucker ist.

2. Polyolinitiator gemäß Anspruch 1, wobei das Molverhältnis der tertiären Dihydroxyaminverbindung zu dem Polyhydroxyalkohol 10 : 1 bis 1 : 10 beträgt.

3. Polyolinitiator gemäß Anspruch 1 oder 2, wobei R¹ Methyl ist und R² und R³ beide Ethyl sind und der Polyhydroxyalkohol Glycerin ist.

4. Eine erste polymere Polyolzusammensetzung, beinhaltend das Reaktionsprodukt von Folgendem:
(a) dem Polyolinitiator gemäß den Ansprüchen 1, 2 oder 3
und
(b) mindestens einer Epoxidverbindung, welche die Struktur X aufweist: 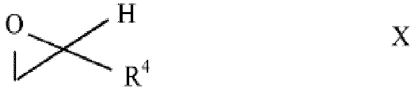 oder
mindestens einer Glycidyletherverbindung, welche die Struktur XI aufweist: 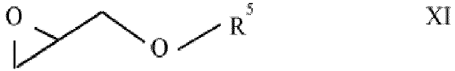 oder einer Kombination davon;
wobei R⁴ Wasserstoff, Phenyl, Cyclohexyl oder ein lineares oder verzweigtes C₁-C₁₈-Alkyl ist
und
R⁵ Wasserstoff, Phenyl, ein lineares oder verzweigtes alkylsubstituiertes C₁-C₆-Phenyl oder ein lineares oder verzweigtes C₁-C₁₈-Alkyl ist.

5. Ein Verfahren zum Herstellen eines Polyurethanpolymers durch die Reaktion einer Mischung, beinhaltend:
(A) eine polymere Polyolformulierung, beinhaltend:
(i) die erste polymere Polyolzusammensetzung gemäß Anspruch 4;
(B) mindestens ein organisches Isocyanat;
(C) optional ein Treibmittel;
und
(D) optional Additive oder Hilfsmittel, die per se für die Produktion von Polyurethanpolymeren bekannt sind.

6. Verfahren gemäß Anspruch 5, wobei die polymere Polyolformulierung (A) ferner Folgendes beinhaltet:
(A)(ii) eine zweite polymere Polyolzusammensetzung, beinhaltend das Reaktionsprodukt von Folgendem:
(c) einem zweiten Polyolinitiator, der durch die folgende Struktur XII dargestellt ist: 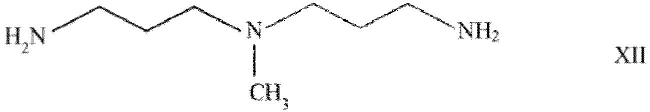 und
(d) mindestens einer Epoxidverbindung, welche die Struktur X aufweist, mindestens einer Glycidyletherverbindung, welche die Struktur XI aufweist, oder einer Kombination davon.

7. Verfahren gemäß Anspruch 5 oder 6, wobei die Reaktion in Gegenwart eines Treibmittels stattfindet und das Polyurethanpolymer in Form eines Polyurethanweichschaumstoffs produziert wird.

## Revendications

1. Une composition d'initiateur polyol comprenant le ou les produits de réaction :
(i) d'un composé aminé tertiaire dihydroxy représenté par la structure I : 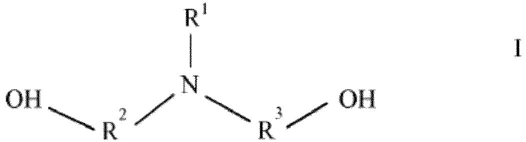
dans laquelle R¹ est un groupe alkyle linéaire ou ramifié en C₁-C₆
et
R² et R³ sont indépendamment un groupe alkyle linéaire ou ramifié en C₁-C₆ et
(ii) d'un alcool polyhydroxy, l'alcool polyhydroxy étant le méthylène glycol, le diéthylène glycol, le méthyl propylène glycol, le dipropylène glycol, le glycérol, le triméthylolpropane, le pentaérythritol, ou un sucre.

2. L'initiateur polyol de la revendication 1 dans lequel le rapport molaire du composé amine tertiaire dihydroxy à l'alcool polyhydroxy va de 10/1 à 1/10.

3. L'initiateur polyol des revendications 1 ou 2 dans lequel R¹ est un méthyle et R² et R³ sont tous les deux un éthyle et l'alcool polyhydroxy est le glycérol.

4. Une première composition de polyol polymère comprenant le produit de réaction :
(a) de l'initiateur polyol des revendications 1, 2 ou 3
et
(b) d'au moins un composé époxyde ayant la structure X : 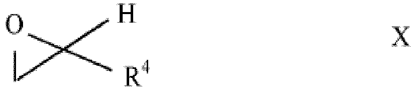 ou
d'au moins un composé d'éther glycidylique ayant la structure XI : 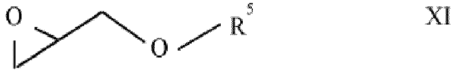 ou d'une combinaison de ceux-ci ;
dans laquelle R⁴ est l'hydrogène, un phényle, un cyclohexyle, ou un alkyle linéaire ou ramifié en C₁-C₁₈
et
R⁵ est l'hydrogène, un phényle, un phényle substitué par un alkyle linéaire ou ramifié en C₁-C₆, ou un alkyle linéaire ou ramifié en C₁-C₁₈.

5. Un procédé afin de réaliser un polymère de polyuréthane par réaction d'un mélange comprenant :
(A) une formulation de polyol polymère comprenant :
(i) la première composition de polyol polymère de la revendication 4 ;
(B) au moins un isocyanate organique ;
(C) facultativement un agent gonflant ;
et
(D) facultativement des additifs ou des agents auxiliaires connus en soi pour la production de polymères de polyuréthane.

6. Le procédé de la revendication 5 dans lequel la formulation de polyol polymère (A) comprend en sus :
(A)(ii) une deuxième composition de polyol polymère comprenant le produit de réaction :
(c) d'un deuxième initiateur polyol représenté par la structure XII suivante : 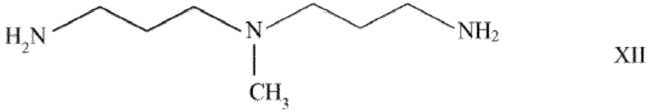 et
(d) d'au moins un composé époxyde ayant la structure X, d'au moins un composé d'éther glycidylique ayant la structure XI, ou d'une combinaison de ceux-ci.

7. Le procédé des revendications 5 ou 6 dans lequel la réaction se produit en présence d'un agent gonflant et le polymère de polyuréthane est produit sous forme d'une mousse souple de polyuréthane.
